# EUROPEAN PATENT SPECIFICATION

(11) **EP 4 000 679 B1**
(45) Date of publication and mention of the grant of the patent: **12.03.2025**
(21) Application number: 21209357.9
(22) Date of filing: 19.11.2021
(51) Int. Cl.: A61M 39/12, A61M 39/22, A61M 5/162, A61J 1/14

(54) **CONNECTOR FOR A BAG CONTAINING LIQUID SOLUTIONS FOR MEDICAL OR NUTRITIONAL USE**
VERBINDUNGSSTÜCK FÜR EINEN BEUTEL, DER FLÜSSIGE LÖSUNGEN FÜR MEDIZINISCHE ODER ERNÄHRUNGSZWECKE ENTHÄLT
CONNECTEUR POUR UN SAC CONTENANT DES SOLUTIONS LIQUIDES À USAGE MÉDICAL OU NUTRITIONNEL

(30) Priority: 23.11.2020 IT 202000028040
(43) Date of publication of application: 25.05.2022
(73) Proprietor: Paolo Gobbi Frattini S.r.l., 20135 Milano (IT)
(72) Inventor: GOBBI FRATTINI, Mr. Paolo Giuseppe, 23035 Sondalo (SO) (IT); CASPANI, Dario, 23033 Grosio (SO) (IT)
(74) Representative: Mittler, Enrico

(56) References cited:
- US-A- 5 776 117
- US-A1- 2011 022 024
- US-A1- 2013 237 946

## Description

The present invention relates to a connector for a bag for containing liquid solutions for medical or nutritional use, in particular a dispensing connector for a bag of the aforesaid type.

In hospitals, nursing homes, retirement homes, and so on, wide use is made of systems for dispensing liquid solutions of drugs or nutrients which, from initial containment bags, enable the liquid solutions to reach the patients' veins.

Dispensing usually takes place through a small tube which extends from the bag in a single piece with the bag itself and a dispensing connector normally closed by a pierceable membrane and having a frangible end part which, for use, is removed to allow the insertion of a piercing terminal placed at the inlet of an infusion set or other appropriate dispensing device.

An important problem relates to the coupling between the small tube and the connector, which is performed by inserting a corresponding tubular part of the connector into the end of the small tube by pressure.

Obviously, the coupling needs to be highly integral so as to prevent any disastrous detachments between the small tube and the connector and ensure a perfect liquid seal, with both properties being capable of lasting over time.

This prerogative is ensured if the sterilization of the bag with the related small tube and connector is performed hot in an autoclave, but this may not be the case with cold sterilization resulting in discomfort and elements of danger for patients and medical personnel.

US 2011/0022024 A1 describes a connector for infusion or transfusion bags which has features similar to those defined in the preamble of claim 1, and in particular it includes a coupling part with two coaxial tubes which define a truncated cone space intended to insert the free end of the small tube leaving the bag.

In view of this state of the art, it has been the object of the present invention to provide a coupling between the small tube and the connector which gives increased integrity and liquid sealing capacities in any use situation and whatever sterilization technique is used.

It was another object to provide a coupling having the aforesaid properties which was usable for any type of connector, for both dispensing and filling the bag.

According to the present invention, such an object has been achieved by the connector defined in claim 1.

The seal against the detachment between small tube and connector and against liquid leaks is thus apparently increased, the forced contact surface between the small tube and the coupling part of the connector being double and lengthened over the entire extension of the annular space with constant section.

Possibly, the sealing capacity can be further increased by associating roughness elements with the inner surface of the outer tube and the outer surface of the inner tube of the coupling part of the connector, which increase the integrity thereof with the tube.

The features of the present invention will become more apparent from the following detailed description of a practical embodiment thereof, shown by way of non-limiting example in the accompanying drawings, in which:
Figure 1 shows a liquid solution bag before coupling it to a dispensing connector according to the present invention;
Figure 2 shows a front view of a connector according to the present invention;
Figure 3 shows a side view of the same connector;
Figure 4 shows said connector in an axial section according to line IV-IV in Figure 2;
Figure 5 shows said connector in an axial section according to line V-V in Figure 3;
Figure 6 shows said connector in a cross section according to line VI-VI in Figure 5;
Figure 7 shows the bag and the connector once the coupling has taken place;
Figure 8 shows an axial section of the coupling obtained between the connector and the small dispensing tube of the bag;
Figure 9 shows a cross-section of said coupling according to the line IX-IX in Figure 8;
Figures 10-12 show the steps of applying an infusion set to the connector in the previous figures.

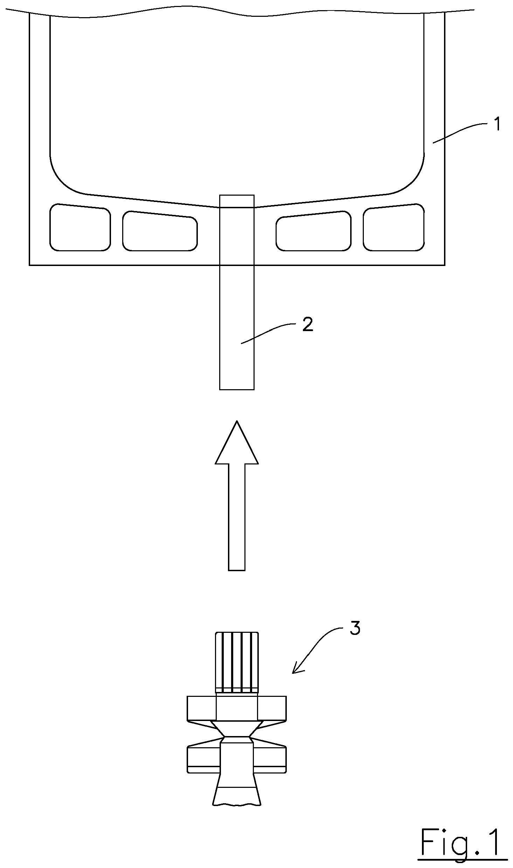

More in particular, Figure 1 shows a flexible liquid solution bag 1 from which a small plastic tube 2 made in a single piece with the bag itself extends outwards.

Before or after filling the bag with a liquid solution, a connector 3 according to the present invention is coupled to the small tube 2, having the task of keeping the small tube itself closed until the time of dispensing the liquid solution by means of an infusion set or other dispensing device applied to the connector 3.

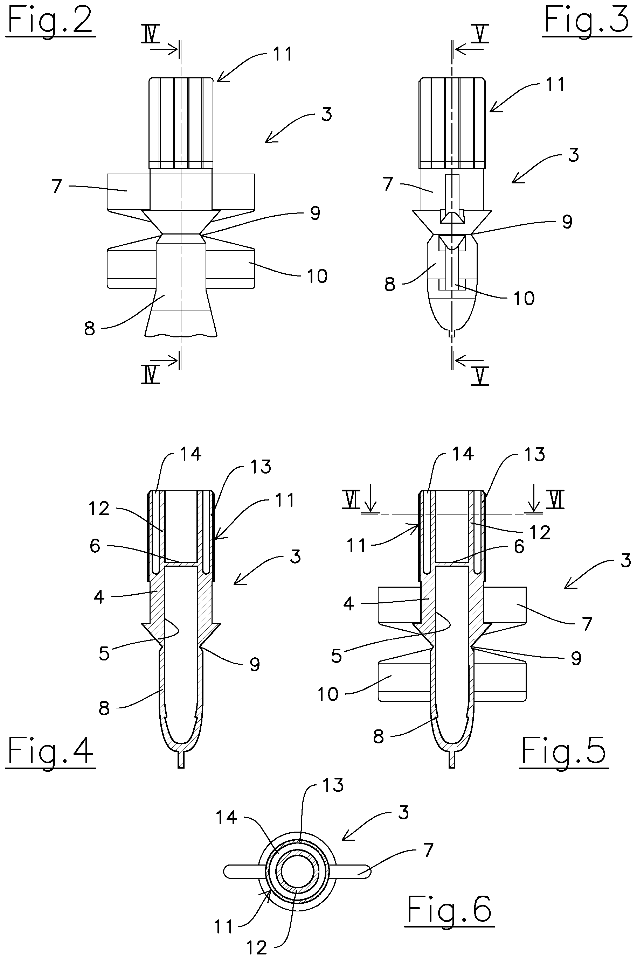

A connector 3 according to the present invention is shown in detail in Figures 2-6 and comprises a single body 4 formed by a coupling part 11 with an axial hole 5 normally closed by a pierceable inner membrane 6 and lateral gripping fins 7 and by a closing part 8 which is detachable from the coupling part 11 at a frangible weakened section 9 and in turn provided with lateral gripping fins 10. Both fins 7 and 10 are positioned close to the weakened section 9.

The coupling part 11, intended to receive the free end of the small tube 2 of bag 1, is formed by two coaxial tubes 12 and 13, inner and outer tubes, respectively, which define an annular space 14 which extends axially with a constant section. Advantageously, the outer surface of the inner tube 12 and the inner surface of the outer tube 13 can have various type of roughness elements, not shown in the drawings.

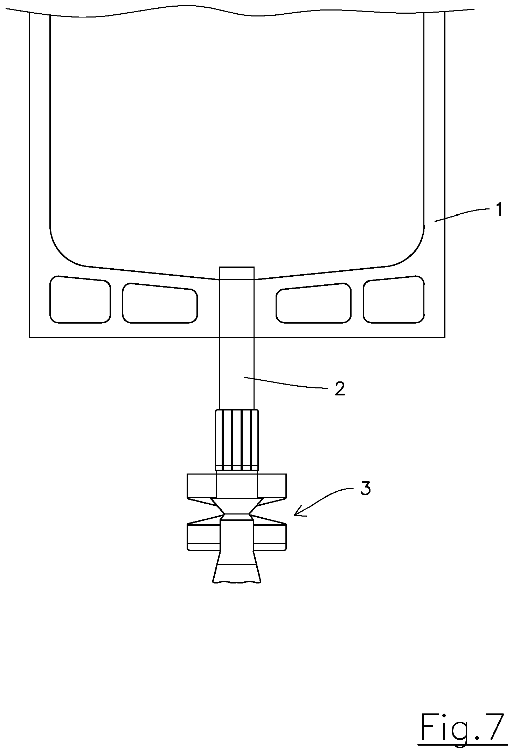

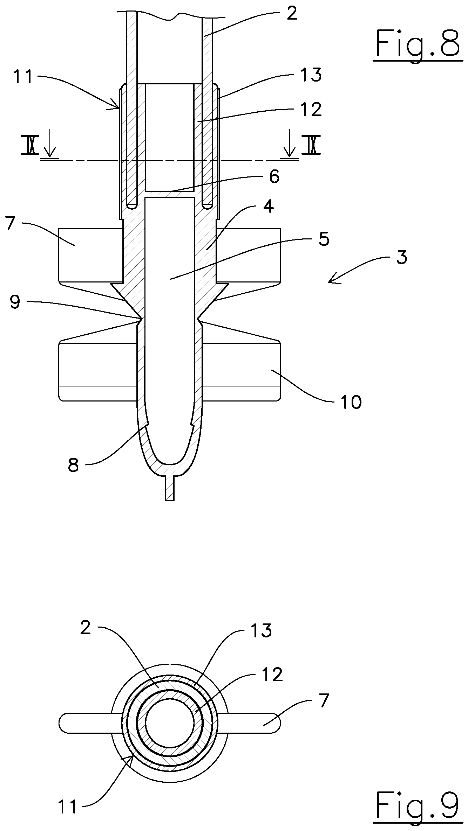

Figure 7 shows the connector 3 coupled to the small dispensing tube 2 of bag 1. The coupling is obtained as shown in Figures 8 and 9, i.e. by inserting the outer end of the small tube 2 forcibly by axial pressure into the annular space 14 between the two coaxial tubes 12 and 13 of the tubular part 11. Any roughness elements provided for the facing surfaces of the two coaxial tubes can increase the integrity between the small tube 2 and the connector 3 and the stability of the coupling thereof.

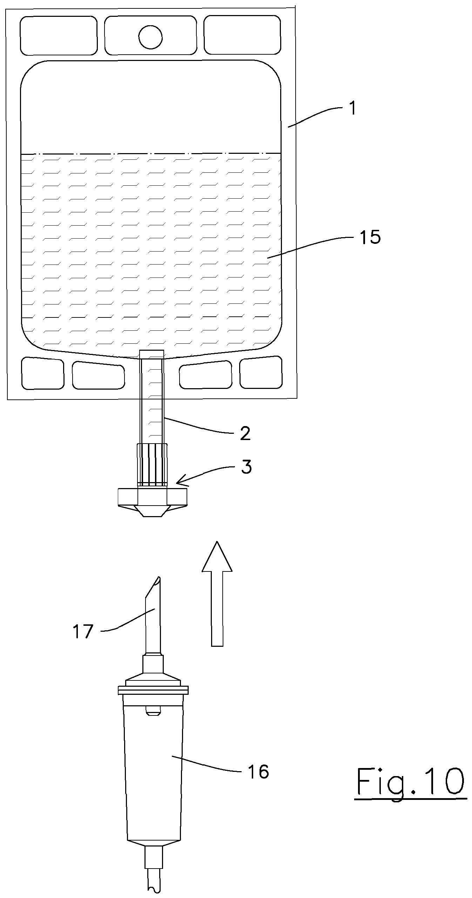

A fluid solution dispensing device can be connected to the bag 1 filled with a fluid solution 15 and having the small dispensing tube 2 and the connector 3 coupled to each other, which device may be of various types, for example an infusion set 16 with an axially hollow piercing terminal 17, as shown in Figure 10.

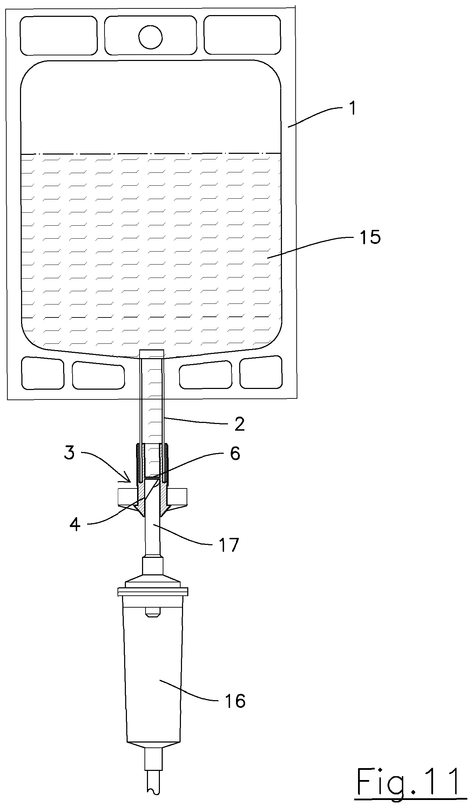

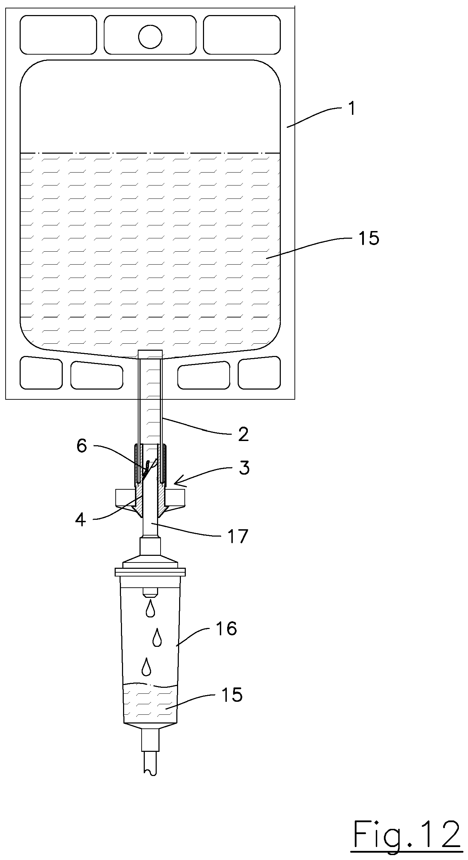

The connection of the infusion set or other dispensing device is carried out after detaching the closing part 8 at the frangible weakened section 9, so that the piercing terminal 17 can be introduced into the axial hole 5 of the main body 4 of connector 3 (Figure 11) up to pierce the closing membrane 6 (Figure 12). The liquid solution 15 can thus flow drop by drop from the bag 1 to the infusion set 16.

It is apparent that the coupling to coaxial tubes described for the dispensing connector 3 of the previous figures can be used for any other type of connector which is associable with a liquid solution bag such as that shown in the drawings. The protection deriving from the present application is thus to be considered as extended to connectors of any type which include a coupling element as defined in the following claims.

## Claims

1. A connector (3) which is applicable to a bag (1) for containing liquid solutions for medical or nutritional use, comprising a coupling part (11) for coupling to an end of a small tube (2) projecting from the bag (1), said coupling part (11) being crossed by an axial hole (5) closed by a pierceable inner membrane (6), and a closing part (8) placed to close the end of said axial hole (5) and detachable from said coupling part (11), wherein said coupling part (11) comprises two coaxial tubes (12, 13) which define an annular inner space (14) therebetween, into which said end of the small tube (2) is forcibly insertable by axial pressure, **characterized in that** said annular inner space (14) extends axially with a constant section along the entire length of said annular space (14), and said coupling part (11) and said closing part (8) form a single integral body (4) provided with a frangible weakened section (9) for the detachment of said closing part (8) from said coupling part (11).

2. A connector (3) according to claim 1, **characterized in that** said single body (4) comprises gripping fins (7, 10) which extend outwards from said coupling part (11) and said closing part (8), respectively, close to said weakened section (9) of the single body (4).

## Patentansprüche

1. Verbindungsstück (3), das auf einen Beutel (1) zum Fassen von flüssigen Lösungen für eine medizinische oder ernährende Verwendung anwendbar ist, das einen Kopplungsteil (11) zum Koppeln an ein Ende eines von dem Beutel (1) vorstehenden kleinen Rohrs (2), wobei der Kopplungsteil (11) von einem mit einer durchbohrbaren inneren Membran (6) verschlossenen axialen Loch (5) gekreuzt wird, und einen Verschließteil (8), der angeordnet ist, das Ende des axialen Lochs (5) zu verschließen, und von dem Kopplungsteil (11) abnehmbar ist, aufweist, wobei der Kopplungsteil (11) zwei koaxiale Rohre (12, 13) aufweist, die dazwischen einen ringförmigen Innenraum (14), in den das Ende des kleinen Rohrs (2) durch einen axialen Druck zwangsweise einbringbar ist, definieren,
**dadurch gekennzeichnet, dass** sich der ringförmige Innenraum (14) mit einem konstanten Querschnitt entlang der gesamten Länge des ringförmigen Raums (14) axial erstreckt, und der Kopplungsteil (11) und der Verschließteil (8) einen integralen Einzelkörper (4) bilden, der mit einem zerbrechbaren geschwächten Abschnitt (9) für das Abnehmen des Verschließteils (8) von dem Kopplungsteil (11) versehen ist.

2. Verbindungsstück (3) gemäß Anspruch 1, **dadurch gekennzeichnet, dass** der Einzelkörper (4) Greifstege (7, 10), die sich von dem Kopplungsteil (11) und dem Verschließteil (8) jeweils nach außen erstrecken, nahe dem geschwächten Abschnitt (9) des Einzelkörpers (4) aufweist.

## Revendications

1. Connecteur (3) qui est applicable à un sac (1) destiné à contenir des solutions liquides à usage médical ou nutritionnel, comprenant une partie de couplage (11) pour se coupler à une extrémité d'un petit tube (2) faisant saillie du sac (1), ladite partie de couplage (11) étant traversée par un trou axial (5) fermé par une membrane interne perforable (6) et une partie de fermeture (8) placée pour fermer l'extrémité dudit trou axial (5) et détachable de ladite partie de couplage (11), dans lequel ladite partie de couplage (11) comprend deux tubes coaxiaux (12, 13) qui définissent un espace interne annulaire (14) entre eux, dans lequel ladite extrémité du petit tube (2) peut être insérée de force par pression axiale, **caractérisé en ce que** ledit espace interne annulaire (14) s'étend de manière axiale avec une section constante le long de toute la longueur dudit espace annulaire (14) et ladite partie de couplage (11) et ladite partie de fermeture (8) forment un seul corps solidaire (4) prévu avec une partie affaiblie cassable (9) pour le détachement de ladite partie de fermeture (8) de ladite partie de couplage (11).

2. Connecteur (3) selon la revendication 1, **caractérisé en ce que** ledit corps (4) unique comprend des ailettes de préhension (7, 10) qui s'étendent vers l'extérieur à partir de ladite partie de couplage (11) et de ladite partie de fermeture (8) respectivement, à proximité de ladite section affaiblie (9) du corps (4) unique.
